# EUROPEAN PATENT SPECIFICATION

(11) **EP 1 659 945 B1**
(45) Date of publication and mention of the grant of the patent: **01.07.2009**
(21) Application number: 04749197.2
(22) Date of filing: 12.08.2004
(51) Int. Cl.: A61B 6/04

(54) **PATIENT REPOSITIONING DEVICE AND METHOD**
VORRICHTUNG UND VERFAHREN ZUR REPOSITIONIERUNG VON PATIENTEN
DISPOSITIF DE REPOSITIONNEMENT D'UN PATIENT

(30) Priority: 28.08.2003 SE 0302303; 03.09.2003 US 499389 P
(43) Date of publication of application: 31.05.2006
(73) Proprietor: Oncolog Medical QA AB, S-756 51 Uppsala (SE)
(72) Inventor: NÄSLUND, Ingemar, S-141 39 Huddinge (SE); LAX, Ingmar, S-113 53 Stockholm (SE)
(74) Representative: Sörby, Lennart
(86) International application number: PCT/SE2004/001186
(87) International publication number: WO 2005/020819

(56) References cited:
- GB-A- 1 290 209
- US-A- 4 592 362
- US-A- 5 983 424

## Description

The present invention relates to a patient repositioning device according to the preamble of claim 1 and a method of repositioning of a patient on a treatment or diagnostic table for radiological procedures according to the preamble of claim 17.

### Background of the Invention:

In radiotherapy as well as in surgical operations, the possibility of locating the actual disease volume with great accuracy is essential for the success of the treatment. To minimize the risk for geographical miss in the treatment, the correct target volume in the body is identified in diagnostic devices such as X-ray systems, Computerized Tomography devices (CT), Gamma Cameras, Nuclear Magnetic Resonance units (NMR or just MR). The responsible physician specifies the target volume to be treated by contours in the diagnostic images and these images are then used for planning of the treatment by manual or computerized methods. The therapeutic procedure, Radiation Therapy or Surgery, is then performed according to the treatment plan. Since the target volume usually is located deep inside the patient body, it is essential that the patient, and especially the target volume, can be repositioned to exactly the same coordinate system as during the diagnostic procedure. This is particularly important in Radiation Therapy of cancer where the intended tumor dose is given as repeated small daily radiation doses during several weeks. It is thus important that in each treatment session the tumor in the patient is correctly positioned relative to the radiation field of the radiotherapy apparatus. Repositioning of patients is also important in diagnostic procedures, particularly when different imaging modalities are used and the images are superimposed to obtain more diagnostic information about the patient.

One prevalent method for positioning a patient's body is that the skin of the part of the body that is to be subjected to radiotherapy is marked with a felt-tip pen, with or without supplementary tattoo points. By means of skin marks and laser position beams in the treatment room, the therapist tries to align the patient in the correct position on the radiotherapy table for each treatment session. The drawback of this method is that the skin is elastic and can move fairly much relative to the skeleton and the target volume. This movement of the skin and the skin marks in relation to the target volume makes it necessary to use wide safety margins on the radiation fields to ensure that the tumor is not outside the radiation field during any of the treatment sessions. The increased size of the radiation fields result in that large volumes of normal tissue without cancer become unnecessarily irradiated. This causes higher doses of radiation energy to the body and leads to a higher risk for undesired side-effects of the radiotherapy. By irradiating greater volumes than necessary, the total radiation dose cannot be increased to the desired level for good curative effect in certain areas, e. g. close to critical organs or tissue with high sensitivity to radiation. A higher accuracy in repositioning of the patient, both during the diagnostic procedure and the treatment, allows a reduction of the safety margin due to position uncertainty and that gives less dose to the healthy tissue and allows a higher dose to the target volume if desired. This in turn gives less risk for side effects and a higher probability for a successful treatment.

A plurality of equipment and techniques are currently available to reduce the deviations in the positioning of a patient, so-called fixation devices such as masks, bite blocks, straps, plastic shells etc. The fixation devices should be usable both with the diagnostic devices, the therapy simulator and the therapy machines with negligible influence to the radiation. The problem with this kind of fixation devices is that the patient who lies down in a shell or is pulled on a horizontal treatment table to get into a table-mounted mask will stretch the skin differently each time, which results in that the skin marks don't keep their position in relation to the target volume. More complex fixations devices with masks that are custom made for each individual patient give a better lateral repositioning, but due to the essentially cylindrical shape of the human body they still permit a fair bit of rotation movement inside the mask, which limits their accuracy. Custom made masks also require significant storage space and systems for safe and certain identification to guarantee that every patient gets the right mask every time.

One device that overcomes some of these problems is disclosed in US 5,983,424. The proposed device is comprised of a non-yielding, upright panel element, a tilting and conveying assembly being arranged at the end of the table for tilting the panel element together with the patient from the upright orientation to a lying orientation and conveying these to a defined place on the table.

The design and function of said device is based on the knowledge that the skin is affected by gravity as the position of the body changes. The thicker the subcutaneous fat, the greater the movements. Also the patient's movement of hips and other parts of the body is difficult to reproduce by today's techniques.

The positioning is thereby performed by carrying out the settings when the patient is placed in an upright position. When a person is standing without clothes and shoes, the skin costume will have a well-defined position in relation to the skeleton. The legs have a given length and the hip-joints will have a position relative to the floor surface that is the same each time.

The skin will be stretched due to gravity, but the stretching will be the same for each session during the radiotherapy period, unless an extreme loss of weight takes place.

The patient stands on a base plate whose rear part is connected to an upright panel behind the patient's back. When the patient takes his natural upright position for the first time, the skin is marked in a suitable position of the body by means of laser position lights or some other technique connected to the panel and indicating a relationship between the back panel and the body. The relationship of the body to the panel then determines the positioning each time.

Various fixation means are then attached to the panel, which like building bricks are snapped onto the panel at various levels, they can be removed after positioning or remain on during diagnostic procedures, simulation work or during radiotherapy. These may involve positioning marks, lateral supports, supports for the curve of the back, head-neck, straps, masks and other fixation aids, connected to the panel.

However, the above procedure of laser positioning of a patient relative to the panel is rather complex and time consuming and the same applies to the attachment of various fixation means to the panel to assure identical repositioning for each treatment in a treatment cycle. Furthermore a complex description of the position of each of the various means attached to the panel is needed to rebuild the supporting arrangement for a special patient, unless the whole panel with the supporting arrangement can be stored in between the treatment sessions, but this requires a large number of repositioning panels at each treatment site, which is expensive and requires a storage system and space for unused panels. If the different support structures instead are individualized or produced in a large number of sizes and different shapes, these support structures have to be stored and handled in an efficient way.

### Summary of the Invention

The object of the present invention is to provide a new patient repositioning device and a method for repositioning of a patient, that overcomes one or more drawbacks of the prior art. This is achieved by the repositioning device as defined in claim 1 and the method according to claim 17.

One advantage with such a device and method is that the procedure of repositioning a patient on a repositioning device is vastly simplified, as the device always centers the patient along the same axis relative to the device.

Another advantage is that one and the same repositioning device can be used for repositioning essentially all patients within a large range of length and width without time consuming adaptations of different support structures.

Still another advantage of this device and method is that essentially no additional support structures of different shapes and sizes are needed, which alleviates both the need for storage space as well as the need for an identification system to ensure that the right type and arrangement of fixation parts is used for each patient.

Embodiments of the invention are defined in the dependent claims.

### Brief Description of the Drawings

The invention will be described in detail below with reference to the drawings, in which:
Fig. 1 is a schematic view of one embodiment of the present invention.
Fig. 2 is another schematic view of one embodiment of the present invention similar to fig 1 illustrating some alternative support arrangements.
Fig 3 is a schematic cross-sectional view of one possible link arrangement along B-B in fig 1.
Fig 4 is a schematic view of another link arrangement.

### Detailed Description of Preferred Embodiments

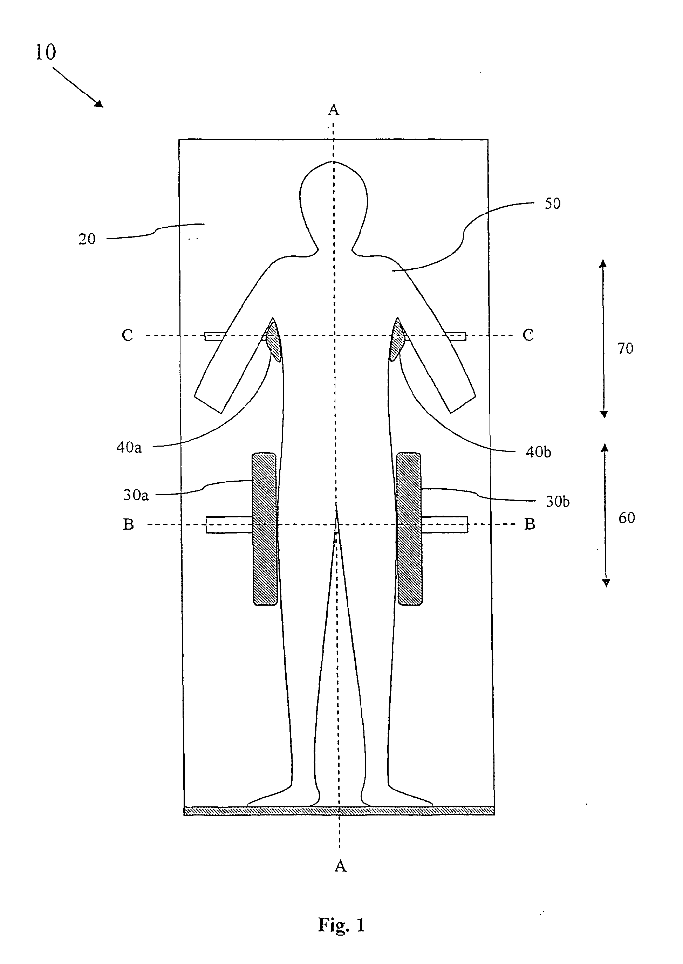

Fig. 1 schematically shows one embodiment of the patient repositioning device 10 according to the present invention. As discussed above, repositioning devices 10 of tilting type presents a large number of advantages compared to other prior art repositioning devices, but the known devices of tilting type tend to be complicated to use.

Like the device disclosed in US 5,983,424, the repositioning device 10 according the present invention comprises a patient supporting panel 20 and at least one pair of laterally (along the broken lines B-B and C-C respectively) adjustable side support structures 30a,b, 40a,b for sideways supporting a patient 50 on the supporting panel 20. But in order to achieve quick and reliable centering of the patient 50 relative the panel 20, each pair of side support structures 30a,b, 40a,b are linked by a link arrangement, which is described below, so that they automatically are centered about a longitudinal axis A-A of the supporting panel indicating the centre of a patient 50 supported on the panel 20.

To achieve reproducible centering of patients 50 each pair of laterally adjustable side support structures 30a,b, 40a,b are arranged to support the patient 50 at a body region with bone structures close to the skin surface, such as the hip region and the chest region. By this general arrangement of the side support structures 30a,b, 40a,b the majority of all patients can be centered on the device with sufficient accuracy for the following diagnostic and treatment procedures.

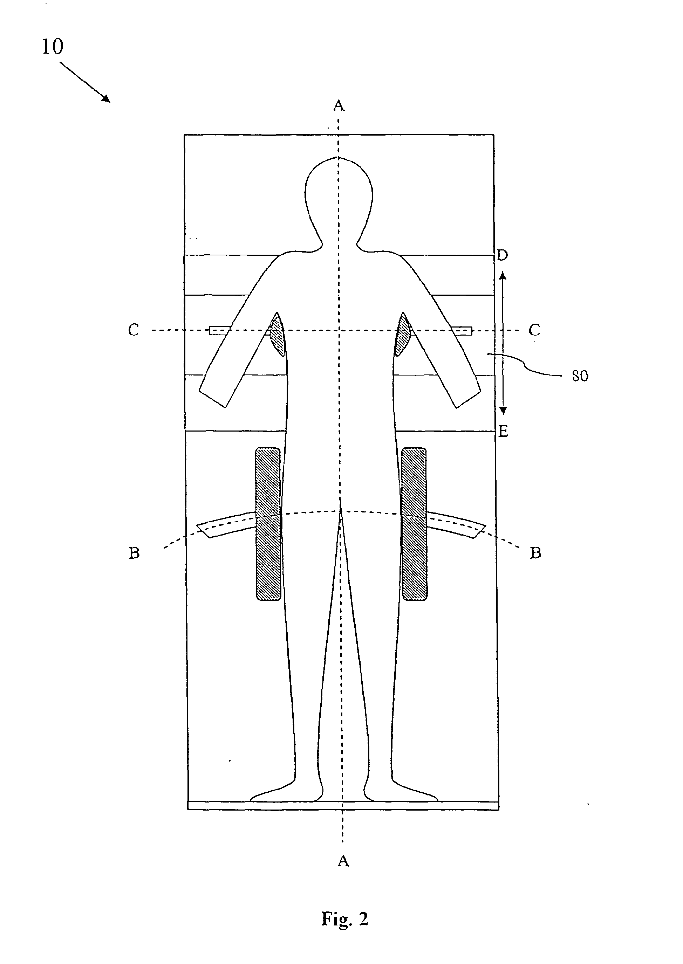

The embodiment shown in fig. 1 comprises two pairs of side support structures 30a,b and 40a,b, a first pair 30a,b (hip support) arranged to support the patient 50 at the hip region 60 and a second pair 40a,b (chest support) arranged to support the patient 50 at the chest region 70. In order to conform with a larger number of patients with respect to their height at least one of the pairs of side support structures 30a,b, 40a,b, preferably both, are longitudinally adjustable, as is indicated for the chest support 40a,b in fig. 2. The chest support 40a,b is mounted on a longitudinally moveable mounting base 80, which is moveable between the positions indicated by D and E in fig. 2. Preferably the mounting base 80 is moveable between a number of fixed positions, so that the base 80 can be arranged in the same position each time a patient is to be treated.

Depending on the type of diagnostic or treatment equipment that the repositioning device 10 is to be used in the device may be comprised of any suitable material. But as the main area of use is radiodiagnostic or radiotherapy essentially all parts located in the upper section of the repositioning device preferably are made of a radiolucent material. However, parts located in regions that do not disturb the diagnostic imaging or treatment may be allowed to be non-radiolucent. One way to avoid having obstructing elements that cannot be made in radiolucent material in the upper section is to arrange such elements outside this section e.g. in or below the feet region. Such obstructing elements are mainly associated with the link arrangement for the support structures 30a,b, 40a,b and will be discussed in greater detail below.

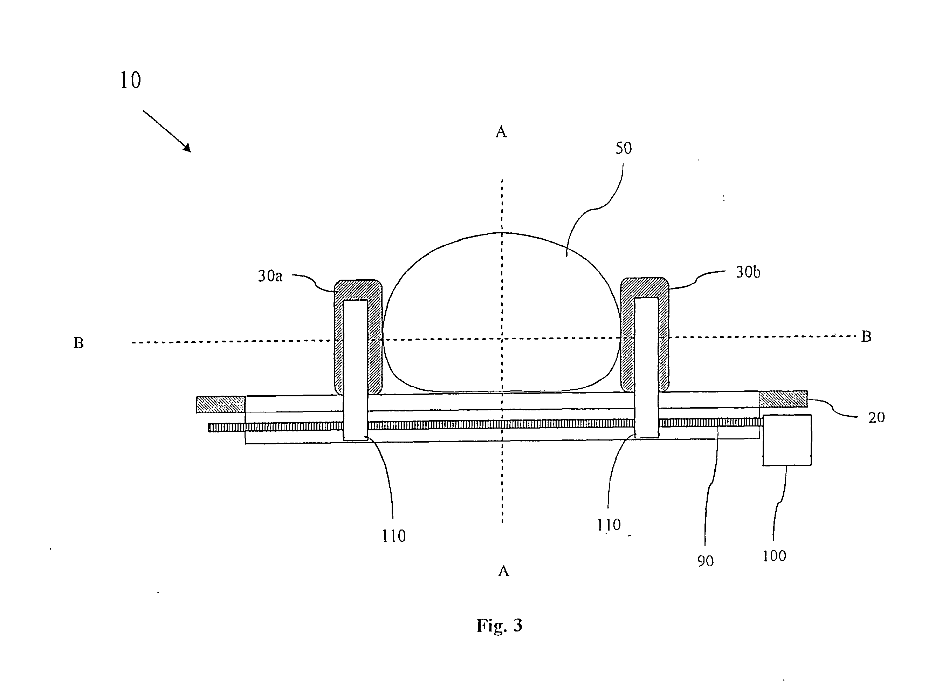

Fig. 3 shows a cross sectional view of the repositioning device 10 with a link arrangement comprising a double-threaded screw 90 to achieve the linked centered behavior of the support structures 30a,b. The double-threaded screw 90 is driven for rotation by a drive motor 100 and the support structures 30a,b are arranged to move apart or together in a synchronized centered manner by threaded portions 110a,b depending on the direction of rotation. There are obviously a number of modifications and variations that may give the same result of a centered motion of the support structures 30a,b, 40a,b, such as two threaded screws rotating in opposite directions through gears or the like. Further, the drive motor 100 can be arranged e.g. at the foot end of the repositioning device 10 and drive the double-threaded screw 90 by a longitudinal axis and an angle gear, whereby the amount of non-radiolucent material can be arranged outside the treatment section of the repositioning device 10. The link arrangement can also be made with two separate motors, each one controlling the position of one side support, and with the motors electronically linked to synchronize their motions to obtain the centered motion of the supports.

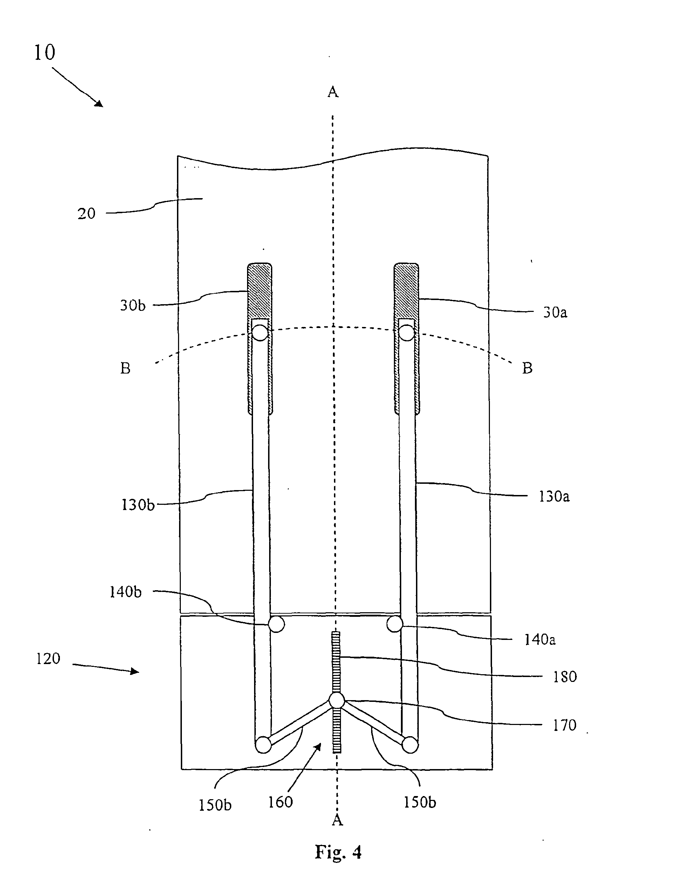

Fig 4 schematically shows another type of link arrangement for the repositioning device 10 according to the present invention wherein the support structures 30a,b are linked by a mechanical lever system 120. The resulting movement of the support structures 30a,b linked by such a lever system is indicated by the line B-B in fig 2. The mechanical lever system 120 is arranged such that only levers 130a,b extends into the treatment section of the repositioning device 10, thereby minimizing the amount of non-radiolucent material in the treatment section. As can be seen in fig. 4 all other parts of the mechanical lever system 120 are arranged at the foot end of the repositioning device 10. The levers 130a,b are pivotally supported at pivotal points 140a,b respectively and are centered and linked together by link levers 150a,b. The link levers 150a,b are pivotally joined by a centering and locking mechanism 160, comprised of a slider 170 and a shaft 180. The centering and locking mechanism 160 ensures that the levers 130a,b always are mutually centered through the link levers 150a,b respectively, and preferably provides one-way automatic lock system that in normal state locks outward movement of the support structures while allowing inward movement to locate a patient. The locking mechanism 160 is preferably released by a release button or the like (not shown). Alternatively, the mechanical lever system 120 may be controlled by a drive motor arranged to drive the shaft 180 for rotation, whereby the shaft 180 and the slider 170 are provided with threads.

In the discussed embodiments the repositioning device 10 has been shown with two pairs of support structures 30a,b, 40a,b, however it may comprise any suitable number of pairs. In one embodiment there is provided a head support in the form of a pair of support structures (head support). To assure that the head is not tilted when it is fixed by the head support, each support may be provided with a sight structure that allows aligning of respective skin markings on the head of the patient. Moreover, additional support may, if needed, be supplied by supplement supports of more conventional type, and the support structures may, if needed, be fitted with support elements of different shape and or size to achieve a close fit for different patients.

The patient supporting panel or any of the support structures may also have alignment means such as reference marks, laser lines or other similar means, to aid in rotational adjustment of the patient against skin marks in different body regions.

The panel 20 of the repositioning device 10 may in conventional ways be fitted with or be fittable with supplementary aids such as visual graduated scales or markers that are visible in diagnostic images to make it possible to position the panel by its own reference coordinate system in relation to equipment in a diagnostic clinic and radiotherapy clinic.

The panel can also be fitted with electronic components such as transceivers, magnets, light sources etc. which can indicate the position in relation to the room and equipment of different types, the position of the patient, the position of the radiation fields on the patient and against the panel or the means of the panel.

Further it is possible to use more than one panel 20 in one treatment session by one panel being arranged behind the patient and one panel (not shown in figures) with accessories being mounted in front of the patient in connection with radiotherapy.

What has been said about the panel 20 does not necessarily signify a flat panel. It can also be shaped like a bucket seat, adapted completely to the contour of the patient, or it may have any appearance whatsoever and it may also be integrated into the treatment table of the diagnostic or therapeutic device.

The support structures may stay on the device to hold the patient in place during the whole procedure and serve as patient fixation, but they may also be removable after the positioning process to allow other means of fixation during the diagnostic or treatment procedure. To further assure that the patient body gets a consistent setup each time, one or several of the side support structures may be provided with a pressure sensor that registers the pressure that the support structure applies on the patient.

Furthermore for easy adjustment of the patient to the intended position, the patient supporting panel can be provided with a low-friction surface or movable panels that lower the friction between the patient body and the patient supporting panel, so that friction cannot cause distorted positioning.

Although the present invention is described in the form of a patient repositioning device of tilting type, the linked patient supporting panels according to the invention may be used for all types of patient repositioning devices where a repeatable positioning of a patient body is desired. For patient repositioning devices of essentially horizontal non tilting type, the patient supporting panel is preferably provided with low-friction surface or movable panels as discussed above.

There is also provided a method of repositioning a patient using a repositioning device 10 according to the present invention. The method comprises the steps:
- arranging the patient with the back, side or front against a patient supporting panel of a patient repositioning device 10,
- sideways repositioning the patient on the supporting panel using at least one pair of laterally adjustable side support structures, the side support structures being linked so that they automatically are centered about a longitudinal axis of the supporting panel ensuring that the patient is essentially identically repositioned about said longitudinal axis every time.

When the repositioning device 10 is provided with longitudinal adjustable support structures the method prior to the step of sideways repositioning compromises the additional step of:
- adjusting the longitudinal position of each pair of side support structures according to individually selected parameters.

When the patient supporting panel or side support structures 30a,b, 40a,b, are provided with alignment means to aid in rotational adjustment of the patient against skin marks in different body regions the method compromises the additional step of:
- adjusting the patient rotation by lining up skin marks with the alignment means.

When patient repositioning device is of tilting type the patient is arranged on the patient supporting panel in an upright position and the method comprises a last step of:
- tilting the patient supporting panel to a predetermined position.

## Claims

1. Patient repositioning device (10) comprising:
a patient supporting panel (20);
at least one pair of laterally adjustable side support structures (30a,b, 40a,b) for sideways supporting a patient (50) on the supporting panel (20)
**characterized in that**
each pair of side support structures (30a,b, 40a,b) are linked by a link arrangement ensuring that the side support structures (30a,b, 40a,b) automatically are centered about a longitudinal axis (A-A) of the supporting panel (20) indicating the centre of a patient (50) supported on the panel (20).

2. Patient repositioning device (10) according to claim 1 **characterized in that** each pair of laterally adjustable side support structures (30a,b, 40a,b) are arranged to support the patient (50) at a body region with bone structures close to the skin surface, such as the hip region (60), the chest region (70) and the head.

3. Patient repositioning device (10) according to claim 1 or 2 **characterized in that** it comprises a first pair of side support structures (30a,b) arranged to support the patient (50) at the hip region (60) and a second pair of side support structures (40a,b) arranged to support the patient (50) at the chest region (70).

4. Patient repositioning device (10) according to any of the claims 1 to 3 **characterized in that** at least one pair of side support structures (30a,b, 40a,b) are longitudinally adjustable.

5. Patient repositioning device (10) according to any of the claims 1 to 4 **characterized in that** essentially all parts located in the treatment section of the repositioning device are radiolucent.

6. Patient repositioning device (10) according to any of the claims 1 to 5 **characterized in that** the link arrangement comprises a one-way automatic lock system.

7. Patient repositioning device (10) according to any of the claims 1 to 6 **characterized in that** the link arrangement comprises a drive motor (100).

8. Patient repositioning device (10) according to any of the claims 1 to 7 **characterized in that** the link arrangement comprises a double-threaded screw (90).

9. Patient repositioning device (10) according to any of the claims 1 to 8 **characterized in that** the link arrangement is comprised of two electronically linked drive motors.

10. Patient repositioning device (10) according to any of the claims 1 to 9 **characterized in that** the link arrangement comprises a mechanical lever system (120).

11. Patient repositioning device (10) according to any of the claims 1 to 10 **characterized in that** the link arrangement comprises at least one non radiolucent part that is arranged outside of the treatment section of the repositioning device, e.g. below the feet support.

12. Patient repositioning device (10) according to any of the claims 1 to 11 **characterized in that** the patient supporting panel and/or one or several of the side support structures (30a,b, 40a,b) are provided with alignment means for adjusting the rotational position of the patient.

13. Patient repositioning device (10) according to any of the claims 1 to 12 **characterized in that** the patient supporting panel (20) is provided with a low-friction surface or movable panels for easy adjustment of the patient (50) to the intended position.

14. Patient repositioning device (10) according to any of the claims 1 to 13 **characterized in that** one or several of the side support structures (30a,b, 40a,b) can be removed from the patient supporting panel (20) after the alignment procedure.

15. Patient repositioning device (10) according to any of the claims 1 to 14 **characterized in that** at least one of the side support structures (30a,b, 40a,b) is provided with a pressure sensor.

16. Patient repositioning device (10) according to any of the claims 1 to 15 **characterized in that** it is of tilting type.

17. Method of repositioning a patient (50) comprising the steps:
arranging the patient (50) with the back, side or front side against a patient supporting panel (20) of a patient repositioning device (10),
sideways repositioning the patient on the supporting panel (20) using at least one pair of laterally adjustable side support structures (30a,b, 40a,b)
**characterized in that**
the side support structures (30a,b, 40a,b) are linked so that they automatically are centered about a longitudinal axis (A-A) of the supporting panel (20) ensuring that the patient (50) is essentially identically repositioned about said longitudinal axis (A-A) every time.

18. Method according to claim 17 **characterized in that** it prior to the step of sideways repositioning comprises the step of:
adjusting the longitudinal position of each pair of side support structures (30a,b, 40a,b) according to individually selected parameters.

19. Method according to claim 17-18 **characterized in that** it also comprises the step of:
adjusting the rotational position of the patient (50) using rotational alignment means.

20. Method according to any of the claims 17 to 19, wherein the patient repositioning device is of tilting type **characterized in that** the patient (50) is arranged on the patient supporting panel (20) in an upright position and the method comprises a last step of:
tilting the patient supporting panel (20) to a predetermined position.

## Patentansprüche

1. Vorrichtung (10) zur Repositionierung von Patienten mit: einer Patientenstützplatte (20);
mindestens einem Paar seitlich einstellbarer Seitenstützstrukturen (30a, b, 40a, b) zum Seitwärtsabstützen eines Patienten (50) auf der Patientenstützplatte (20),
**dadurch gekennzeichnet, daß**
jedes Paar Seitenstützstrukturen (30a, b, 40a, b) durch eine Koppelanordnung gekoppelt ist, die gewährleistet, daß die Seitenstützstrukturen (30a, b, 40a, b) um eine Längsachse (A-A) der Stützplatte (20), die die Mitte eines auf der Platte (20) abgestützten Patienten (50) angibt, automatisch zentriert sind.

2. Vorrichtung (10) zur Repositionierung von Patienten nach Anspruch 1, **dadurch gekennzeichnet, daß** jedes Paar seitlich einstellbare Seitenstützstrukturen (30a, b, 40a, b) so angeordnet ist, daß es den Patienten (50) an einer Körperregion mit Knochenstrukturen nahe der Hautoberfläche abstützt, z. B. an der Hüftregion (60), an der Brustkorbregion (70) und am Kopf.

3. Vorrichtung (10) zur Repositionierung von Patienten nach Anspruch 1 oder 2, **dadurch gekennzeichnet, daß** sie aufweist: ein erstes Paar Seitenstützstrukturen (30a, b), das so angeordnet ist, daß es den Patienten (50) an der Hüftregion (60) abstützt, und ein zweites Paar Seitenstützstrukturen (40a, b), das so angeordnet ist, daß es den Patienten (50) an der Brustkorbregion (70) abstützt.

4. Vorrichtung (10) zur Repositionierung von Patienten nach einem der Ansprüche 1 bis 3, **dadurch gekennzeichnet, daß** mindestens ein Paar Seitenstützstrukturen (30a, b, 40a, b) längs einstellbar ist.

5. Vorrichtung (10) zur Repositionierung von Patienten nach einem der Ansprüche 1 bis 4, **dadurch gekennzeichnet, daß** im wesentlichen alle im Behandlungsabschnitt der Vorrichtung zur Repositionierung liegenden Teile strahlendurchlässig sind.

6. Vorrichtung (10) zur Repositionierung von Patienten nach einem der Ansprüche 1 bis 5, **dadurch gekennzeichnet, daß** die Koppelanordnung ein automatisches Einweg-Arretiersystem aufweist.

7. Vorrichtung (10) zur Repositionierung von Patienten nach einem der Ansprüche 1 bis 6, **dadurch gekennzeichnet, daß** die Koppelanordnung einen Antriebsmotor (100) aufweist.

8. Vorrichtung (10) zur Repositionierung von Patienten nach einem der Ansprüche 1 bis 7, **dadurch gekennzeichnet, daß** die Koppelanordnung eine Doppelgewindeschraube (90) aufweist.

9. Vorrichtung (10) zur Repositionierung von Patienten nach einem der Ansprüche 1 bis 8, **dadurch gekennzeichnet, daß** die Koppelanordnung zwei elektronisch gekoppelte Antriebsmotoren aufweist.

10. Vorrichtung (10) zur Repositionierung von Patienten nach einem der Ansprüche 1 bis 9, **dadurch gekennzeichnet, daß** die Koppelanordnung ein mechanisches Hebelsvstem (120)

11. Vorrichtung (10) zur Repositionierung von Patienten nach einem der Ansprüche 1 bis 10, **dadurch gekennzeichnet, daß** die Koppelanordnung mindestens ein nicht strahlendurchlässiges Teil aufweist, das außerhalb des Behandlungsabschnitts der Vorrichtung zur Repositionierung angeordnet ist, z. B. unter der Fußstütze.

12. Vorrichtung (10) zur Repositionierung von Patienten nach einem der Ansprüche 1 bis 11, **dadurch gekennzeichnet, daß** die Patientenstützplatte und/oder eine oder mehrere der Seitenstützstrukturen (30a, b, 40a, b) mit einer Justiereinrichtung zum Einstellen der Drehposition des Patienten versehen sind.

13. Vorrichtung (10) zur Repositionierung von Patienten nach einem der Ansprüche 1 bis 12, **dadurch gekennzeichnet, daß** die Patientenstützplatte (20) mit einer reibungsarmen Oberfläche oder beweglichen Platten zur leichten Einstellung des Patienten (50) in der beabsichtigten Position versehen ist.

14. Vorrichtung (10) zur Repositionierung von Patienten nach einem der Ansprüche 1 bis 13, **dadurch gekennzeichnet, daß** eine oder mehrere der Seitenstützstrukturen (30a, b, 40a, b) nach dem Justierverfahrensablauf von der Patientenstützplatte (20) entfernt werden können.

15. Vorrichtung (10) zur Repositionierung von Patienten nach einem der Ansprüche 1 bis 14, **dadurch gekennzeichnet, daß** mindestens eine der Seitenstützstrukturen (30a, b, 40a, b) mit einem Drucksensor versehen ist.

16. Vorrichtung (10) zur Repositionierung von Patienten nach einem der Ansprüche 1 bis 15, **dadurch gekennzeichnet, daß** sie vom Kipp-Typ ist.

17. Verfahren zur Repositionierung eines Patienten (50) mit den Schritten:
Anordnen des Patienten (50) mit dem Rücken, der Seite oder der Vorderseite an einer Patientenstützplatte (20) einer Vorrichtung (10) zur Repositionierung von Patienten,
Seitwärtsrepositionieren des Patienten auf der Stützplatte (20) mit Hilfe mindestens eines Paars seitlich einstellbarer Seitenstützstrukturen (30a, b, 40a, b),
**dadurch gekennzeichnet, daß**
die Seitenstützstrukturen (30a, b, 40a, b) so gekoppelt sind, daß sie um eine Längsachse (A-A) der Stützplatte (20) automatisch zentriert werden, was gewährleistet,
daß der Patient (50) um die Längsachse (A-A) jedesmal im wesentlichen identisch repositioniert wird.

18. Verfahren nach Anspruch 17, **dadurch gekennzeichnet, daß** es vor dem Schritt des Seitwärtspositionierens den Schritt aufweist:
Einstellen der Längsposition jedes Paars Seitenstützstrukturen (30a, b, 40a, b) in Übereinstimmung mit individuell ausgewählten Parametern.

19. Verfahren nach Anspruch 17 oder 18, **dadurch gekennzeichnet, daß** es ferner den Schritt aufweist:
Einstellen der Drehposition des Patienten (50) mit Hilfe einer Drehjustiereinrichtung.

20. Verfahren nach einem der Ansprüche 17 bis 19, wobei die Typ ist, **dadurch gekennzeichnet, daß** der Patient (50) auf der Patientenstützplatte (20) in einer aufrechten Position angeordnet wird und das Verfahren als letzten Schritt aufweist:
Kippen der Patientenstützplatte (20) in eine vorbestimmte Position.

## Revendications

1. Dispositif de repositionnement d'un patient (10), comprenant :
un panneau de soutien d'un patient (20) ;
au moins une paire de structures de soutien latérales réglables latéralement (30a,b, 40a,b) pour soutenir un patient (50) sur les côtés, sur le panneau de soutien (20)
**caractérisé en ce que**
chaque paire de structures de soutien latérales (30a,b, 40a,b) est liée par un dispositif de liaison assurant que les structures de soutien latérales (30a,b, 40a,b) sont automatiquement centrées par rapport à un axe longitudinal (A-A) du panneau de soutien (20) indiquant le centre du patient (50) soutenu sur le panneau (20).

2. Dispositif de repositionnement d'un patient (10) selon la revendication 1, **caractérisé en ce que** chaque paire de structures de soutien latérales réglables latéralement (30a,b, 40a,b) est disposée de façon à soutenir le patient (50) au niveau d'une zone corporelle ayant des structures osseuses proches de la surface de la peau, comme la région de la hanche (60), la région du thorax (70) et la tête.

3. Dispositif de repositionnement d'un patient (10) selon la revendication 1 ou 2, **caractérisé en ce qu'**il comprend une première paire de structures de soutien latérales (30a,b) disposée de façon à soutenir le patient (50) au niveau de la zone de la hanche (60) et une deuxième paire de structures de soutien latérales (40a,b) disposée de façon à soutenir le patient (50) dans la zone du thorax (70).

4. Dispositif de repositionnement d'un patient (10) selon l'une quelconque des revendications 1 à 3, **caractérisé en ce qu'**au moins une paire de structures de soutien latérales (30a,b, 40a,b) est réglable longitudinalement.

5. Dispositif de repositionnement d'un patient (10) selon l'une quelconque des revendications 1 à 4, **caractérisé en ce qu'**essentiellement toutes les parties situées dans la section de traitement du dispositif de repositionnement sont radiotransparentes.

6. Dispositif de repositionnement d'un patient (10) selon l'une quelconque des revendications 1 à 5, **caractérisé en ce que** le dispositif de liaison comprend un système de verrouillage automatique unidirectionnel.

7. Dispositif de repositionnement d'un patient (10) selon l'une quelconque des revendications 1 à 6, **caractérisé en ce que** le dispositif de liaison comprend un moteur d'entraînement (100).

8. Dispositif de repositionnement d'un patient (10) selon l'une quelconque des revendications 1 à 7, **caractérisé en ce que** le dispositif de liaison comprend une vis à double filetage (90).

9. Dispositif de repositionnement d'un patient (10) selon l'une quelconque des revendications 1 à 8, **caractérisé en ce que** le dispositif de liaison comprend deux moteurs d'entraînement reliés électroniquement.

10. Dispositif de repositionnement d'un patient (10) selon l'une quelconque des revendications 1 à 9, **caractérisé en ce que** le dispositif de liaison comprend un système de levier mécanique (120).

11. Dispositif de repositionnement d'un patient (10) selon l'une quelconque des revendications 1 à 10, **caractérisé en ce que** le dispositif de liaison comprend au moins une partie radiotransparente, qui est disposée hors de la section de traitement du dispositif de repositionnement, par exemple sous le support à pied.

12. Dispositif de repositionnement d'un patient (10) selon l'une quelconque des revendications 1 à 11, **caractérisé en ce que** le panneau de soutien du patient et/ou une ou plusieurs des structures de soutien latérales (30a,b, 40a,b) sont dotés de moyens d'alignement permettant d'ajuster la position en rotation du patient.

13. Dispositif de repositionnement d'un patient (10) selon l'une quelconque des revendications 1 à 12, **caractérisé en ce que** le panneau de soutien du patient (20) est doté d'une surface à faible frottement ou de panneaux mobiles pour un ajustement facile du patient (50) dans la position souhaitée.

14. Dispositif de repositionnement d'un patient (10) selon l'une quelconque des revendications 1 à 13, **caractérisé en ce qu'**une ou plusieurs des structures de soutien latérales (30a,b, 40a,b) peuvent être enlevées du panneau de soutien du patient (20) après la procédure d'alignement.

15. Dispositif de repositionnement d'un patient (10) selon l'une quelconque des revendications 1 à 14, **caractérisé en ce qu'**au moins une des structures de support latérales (30a,b, 40a,b) est dotée d'un capteur de pression.

16. Dispositif de repositionnement d'un patient (10) selon l'une quelconque des revendications 1 à 15, **caractérisé en ce qu'**il est de type inclinable.

17. Procédé de repositionnement d'un patient (50) comprenant les étapes consistant à :
disposer le patient (50) avec le dos, le côté ou la partie avant contre un panneau de soutien du patient (20) d'un dispositif de repositionnement d'un patient (10),
repositionner latéralement le patient sur le panneau de soutien (20) en utilisant au moins une paire de structures de soutien latérales réglables latéralement (30a,b, 40a,b)
**caractérisé en ce que**
les structures de soutien latérales (30a,b, 40a,b) sont liées de sorte qu'elles soient automatiquement centrées par rapport à un axe longitudinal (A-A) du panneau de soutien (20), assurant que le patient (50) est repositionné essentiellement de manière identique par rapport audit axe longitudinal (A-A) à chaque fois.

18. Procédé selon la revendication 17, **caractérisé en ce qu'**avant l'étape de repositionnement latéral, il comprend l'étape consistant à :
ajuster la position longitudinale de chaque paire de structures de soutien latérales (30a,b, 40a,b) selon des paramètres choisis individuellement.

19. Procédé selon les revendications 17-18, **caractérisé en ce qu'**il comprend également l'étape consistant à :
ajuster la position en rotation du patient (50) en utilisant des moyens d'alignement en rotation.

20. Procédé selon l'une quelconque des revendications 17 à 19, dans lequel le dispositif de repositionnement du patient est de type inclinable, **caractérisé en ce que** le patient (50) est disposé sur le panneau de soutien du patient (20) dans une position verticale et le procédé comprend une dernière étape consistant à :
incliner le panneau de soutien du patient (20) dans une position prédéterminée.
